# FASCICULE DE BREVET EUROPEEN

(11) **EP 0 311 496 B1**
(45) Date de publication et mention de la délivrance du brevet: **26.01.1994**
(21) Numéro de dépôt: 88402494.4
(22) Date de dépôt: 03.10.1988
(51) Int. Cl.: C12P 19/04, A01G 33/00

(54) **Procédé de production et d'extraction de polysaccharides à partir d'une culture de porphyridium cruentum et dispositif pour la mise en oeuvre du procédé**
Verfahren zur Herstellung und Extraktion von Polysacchariden aus einer Kultur von Porphyridium cruentum und Vorrichtung, um dieses Verfahren auszuführen
Process for the preparation and extraction of polysaccharides from a culture of phorphyridium cruentum and apparatus for carrying out this process

(30) Priorité: 06.10.1987 FR 8713780
(43) Date de publication de la demande: 12.04.1989
(73) Titulaire: COMMISSARIAT A L'ENERGIE ATOMIQUE, 75015 Paris Cédex 15 (FR)
(72) Inventeur: Barnier, Henri, F-13100 Aix en Provence (FR); Ferreira Dos Santos, Patrick, F-13770 Venelles (FR); Gudin, Claude, F-13100 Aix en Provence (FR); Thepenier, Catherine, F-04100 Manosque (FR)
(74) Mandataire: Mongrédien, André

(56) Documents cités:
- US-A- 4 087 936
- MIRCEN JOURNAL OF APPLIED MICROBIOLOGY AND BIOTECHNOLOGY, vol. 1, 1985, pages 257-268, Oxford University Press in association with UNESCO; C. THEPENIER et al.: "Studies on optimal conditions for polysaccharide production by Porphyridium cruentum"
- RAPPORT EUR 9975 FR de la Commission des Communautés européennes: Energie, 1985, pages 20-29; C. GUDIN et al.: "Etude et développement de photo-réacteurs tubulaires pour la production maitrisée de biomasse cellulaire photosynthétique "methanisable" et de biomasse exocellulaire spécifique"
- BIOTECHNOLOGY AND BIOENGINEERING SYMP., no. 15, 1985, pages 533-547, John Wiley & Sons Inc.; D.B. ANDERSON et al.: "A process for the production of polysaccharides from microalgae"

## Description

La présente invention a pour objet un procédé de production et d'extraction de polysaccharides à partir d'une culture de microalgues dans un photobioréacteur solaire ainsi que le photobioréacteur pour la mise en oeuvre de ce procédé. La microalgue étudiée est Porphyridium cruentum qui excrète des polysaccharides formant une gangue autour de la membrane cellulaire dont l'épaisseur varie avec l'âge de la cellule.

Les polysaccharides produits par Porphyridium cruentum sont de trois types : les polysaccharides intracellulaires, les polysaccharides péricellulaires et les polysaccharides hydrosolubilisés. Ces trois types de polysaccharides présentent un poids moléculaire supérieur à 1 000 000 de Dalton pouvant aller jusqu'à 4 000 000 de Dalton et comportent les mêmes produits de base, c'est-à-dire des sucres, des sulfates et des acides uroniques.

Les sucres sont principalement le glucose, le galactose et le xylose.

La proportion de chacun des produits de base dans les trois types de polysaccharides n'est pas connue. Quant aux caractéristiques rhéologiques, elles sont identiques pour les polysaccharides péricellulaires et hydrosolubilisés et méconnues pour les polysaccharides intracellulaires.

Le procédé selon l'invention a pour objet la production et l'extraction des polysaccharides hydrosolubilisés seuls ainsi que la production et l'extraction simultanées des trois types de polysaccharides.

L'invention s'applique avantageusement dans le domaine de l'agro-alimentaire et dans celui de l'extraction du pétrole. Dans le domaine agro-alimentaire, les polysaccharides sont employés comme additifs pour modifier la viscosité d'un milieu aqueux ou laiteux et sont utilisés en particulier en charcuterie ou dans les crèmes glacées. Dans le domaine pétrolier, les polysaccharides sont utilisés comme agents tensio-actifs pour modifier le rapport de forces avec la roche magasin et permettre ainsi l'extraction du pétrole de la roche.

Il est connu par le brevet US-A-3 195 271 de cultiver artificiellement Porphyridium cruentum pour produire des polysaccharides. Le procédé décrit dans ce brevet préconise l'addition d'alcool à la culture de microalgue, dont le rapport volume d'alcool/volume de culture est égal à 1, pour former un coagulum. Le volume de culture, traité à l'alcool, est prélevé directement du cultivateur solaire sans être soumis à une étape de concentration.

En outre, ce procédé ne prévoit pas l'extraction ou la purification des polysaccharides ni la récupération des microalgues ou biomasse en vue d'une nouvelle production de polysaccharides.

Comme procédé de production et d'extraction des polysaccharides de Porphyridium cruentum, on peut citer celui décrit dans le brevet US-A-4 417 415. Ce procédé traite de la récupération des polysaccharides totaux, c'est-à-dire intra, péri et exocellulaires. Il comporte une étape de concentration par ultrafiltration suivie d'une basification à chaud du concentrat avec de la soude puis une acidification à froid avec de l'acide chlorhydrique. Cette hydrolyse est suivie d'une filtration puis d'une première précipitation à l'éthanol en utilisant 2 à 3 volumes d'éthanol par volume de filtrat.

Le précipité obtenu est ensuite redissous dans une solution de chlorure de calcium. Une seconde précipitation à l'éthanol avec 3 volumes d'éthanol par volume de solution est alors effectuée. Ce deuxième précipité constitué essentiellement de polysaccharides est séché puis broyé.

Ce procédé entraîne nécessairement la destruction de la biomasse et donc la production d'une nouvelle biomasse à chaque cycle de production de polysaccharides. En plus de la destruction de la biomasse, il présente comme autre inconvénient d'être long et fastidieux à mettre en oeuvre. En outre, les quantités d'alcool utilisées sont très importantes, entraînant un séchage long et coûteux. De plus, les filtrats traités par la soude et l'acide chlorhydrique sont peu concentrés en polysaccharides et les quantités nécessaires de ces deux produits sont importantes augmentant encore le coût du procédé.

Un procédé voisin de production et d'extraction de polysaccharides totaux par culture de Porphypidium cruentum utilisant aussi une étape de concentration suivie d'une basification à chaud puis d'une acidification est décrit dans le document Biotechnology and Bioengineering Symp. n°15(1985) pages 533-547, D.B. Anderson et al ; "a process for the production of polysaccharides from microalgae".

D'autres procédés, tels que celui décrit dans le document US-A-4 087 936, sont relatifs à la production de biopolymères totaux fabriqués par Porphyridium aerugineum. Là encore la biomasse est détruite.

La présente invention a justement pour objet un procédé de production et d'extraction des polysaccharides produits par Porphyridium cruentum relativement simple et peu coûteux avec récupération et valorisation de la biomasse.

De façon plus précise, l'invention a pour objet un procédé de production et d'extraction de polysaccharides à partir d'une culture de la microalgue Porphyridium cruentum, comprenant une étape de concentration des polysaccharides péri- et exocellulaires produits par la microalgue, une étape de séparation des phases solide et liquide en présence en vue du recyclage de la microalgue, une étape de chauffage à une température allant de 60 à 100°C, une étape de précipitation des polysaccharides présents dans la phase liquide et une étape de séchage du précipité obtenu.

Le procédé selon l'invention contrairement à celui décrit dans le brevet US-A-4 417 415 et l'article de B.D. Anderson ne porte que sur les polysaccharides péri et exocellulaires, c'est-à-dire sur les polysaccharides excrétés par la microalgue et formant une gangue autour de la membrane cellulaire. Ces polysaccharides représentent 70 à 75% des polysaccharides totaux, ce qui est largement suffisant et ne justifie pas les procédés longs et coûteux de l'art antérieur pour l'extraction de tous les polysaccharides, y compris ceux présents dans les microalgues.

Ainsi, la phase solide contenant les microalgues peut être recyclée en vue d'une nouvelle production et extraction de polysaccharides contrairement à l'art antérieur. Aussi le procédé de l'invention comprend avantageusement une étape de séparation des phases solide et liquide en présence. En outre, il ne comporte pas d'étape de basification ni d'étape de neutralisation.

Le procédé de l'invention comprend en outre une étape de pressage du précipité de polysaccharides, juste avant le séchage du précipité.

L'étape de chauffage selon l'invention permet une augmentation d'au moins 18% des matières précipitables, pouvant aller jusqu'à 50%, entraînant une augmentation des polysaccharides extraits et purifiés de 30% au moins (pouvant aller jusqu'à 80%) par rapport à un procédé ne comportant pas cette étape de chauffage. Cette étape permet en fait une solubilisation partielle des polysaccharides de la gangue fabriquée par la microalgue.

De préférence, cette étape de chauffage est effectuée à une température de l'ordre de 80°C. La durée de ce chauffage varie de 30 à 60 minutes.

Selon l'invention, l'étape de précipitation peut être réalisée avant ou après l'étape de concentration. Il en est de même pour l'étape de séchage du précipité.

Par ailleurs, l'étape de chauffage peut être effectuée avant ou après l'étape de concentration. De même, elle peut être réalisée avant ou après l'étape de séparation des phases solide et liquide.

L'ordre des étapes du procédé dépend de la nature des étapes.

La précipitation des polysaccharides selon l'invention peut être réalisée avec du chlorure de cétyl-pyridinium noté CPC, cette précipitation par un sel d'ammonium quaternaire étant rendue possible grâce au caractère polyanionique (groupement COO⁻ et SO₃⁻ ) des polysaccharides.

L'addition d'une quantité suffisante de CPC dans la solution polysaccharidique entraîne la formation d'un précipité de composition fixe, formé de 55% de polysaccharides et 45% de CPC.

Cette technique de précipitation permet une concentration des polysaccharides d'un facteur supérieur à 5.

Etant donné les difficultés actuelles pour éliminer totalement l'ammonium résiduel, la précipitation des polysaccharides présents dans la phase liquide est de préférence réalisée par addition d'alcool dans un rapport R volume d'alcool/volume de la phase liquide juste avant d'ajouter l'alcool au plus égal à 1 et en particulier allant de 0,5 à 1.

Avec le procédé selon l'invention, il est possible d'extraire plus de 90% de la matière organique contenue dans la phase liquide en n'utilisant que 50% en volume d'alcool, ce qui correspond à un rapport égal à 1.

Cette méthode de précipitation, bien que moins sélective que celle par le CPC, la quantité de matière récoltée par le CPC ne représentant que 40% des matières organiques précipitées par l'alcool, est beaucoup plus simple que la précipitation par le CPC.

La précipitation par l'alcool s'accompagne d'une coprécipitation saline, le précipité obtenu contenant 70 à 80% de sels.

La masse des précipités obtenus est de 5 à 10% plus élevée lorsque l'addition d'alcool est rapide par rapport à une addition lente. En outre, ces précipités ne contiennent plus que 40% de sels après six lavages dans des mélanges hydroalcooliques.

Les alcools utilisables dans l'invention sont des alcools primaires, secondaires ou tertiaires ayant de 1 à 3 atomes de carbone. Comme alcools utilisables, on peut citer le méthanol, l'éthanol, l'isopropanol, le n-propanol et un mélange de ceux-ci.

Selon l'invention, l'étape de concentration des polysaccharides est réalisée soit par écrémage, soit par ultrafiltration éventuellement associée à une diafiltration.

La récolte par écrémage des polysaccharides exocellulaires est réalisée en transférant la culture, en fin de cycle cultural, dans un photobioréacteur exposé à la lumière solaire, en conditions stagnantes, entraînant la formation en surface d'une crème riche en polysaccharides que l'on récupère par écrémage.

A l'analyse, le concentrat crémeux obtenu se révèle riche en polysaccharides, la teneur étant nettement supérieure à celle de la culture sous-jacente. Après prélèvement de ce concentrat, on constate sa reconstitution dans les heures qui suivent.

La formation du concentrat crémeux est un phénomène de surface. En effet, aucune évolution de la viscosité ni de la teneur en polysaccharides n'est mise en évidence dans la culture sous-jacente et ce, quel que soit le volume de cette culture.

La crème présente l'aspect d'un voile épais et visqueux contenant de 5 à 40 g/l de polysaccharides hydrosolubilisés, ce qui représente par rapport à la culture sous-jacente un facteur de concentration de 10 à 60 de la teneur en polysaccharides hydrosolubilisés. Elle contient les trois types de polysaccharides définis précédemment.

Par solubilisation de celle-ci dans de l'eau chauffée entre 60 et 100°C, on obtient une solution concentrée uniformément dispersée. La récolte des polysaccharides hydrosolubilisés, par séparation des phases solide et liquide par centrifugation ou filtration, nécessite une dilution préalable de cette solution.

Le "moteur" de cette crème est la lumière solaire. En outre, ce phénomène peut être favorisé en maintenant le photobioréacteur à une température allant de 10 à 40°C, la durée de présence du milieu de culture dans le photobioréacteur variant de 12 à 72 heures et de préférence de 24 à 48 heures.

Par ailleurs, la quantité de crème formée est liée au rapport surface/volume du photobioréacteur.

Aussi, ce dernier présente avantageusement la forme d'une cuve "plate" dont le rapport surface/volume est au moins égal à 10 m⁻¹, avec une hauteur minimale de 10 cm.

La concentration des polysaccharides peut aussi être réalisée par ultrafiltration associée de préférence à une diafiltration.

L'ultrafiltration ou diafiltration est réalisée de façon classique. Lors de la diafiltration le liquide introduit est soit de l'eau, ce qui correspond à une diafiltration simple, soit un milieu de salinité définie, ce qui correspond à une diafiltration avec changement de balance ionique.

Les concentrats obtenus présentent les mêmes caractéristiques rhéologiques que les solutions natives (pseudo plasticité, seuil d'écoulement, faible sensibilité aux sels) et leur viscosité est nettement supérieure à celle des solutions natives.

La diafiltration qui est notamment associée à l'ultrafiltration a pour but de diminuer la charge saline totale des concentrats soit en gardant les mêmes proportions de sels, soit en modifiant la composition saline par exemple en échangeant les ions Ca⁺⁺ avec les ions Na⁺ .

La modification du rapport Na⁺ /Ca⁺⁺ (par élimination des ions Na⁺) permet de précipiter la totalité des polysaccharides sous forme calcique, par la technique de précipitation alcoolique avec un taux d'alcool de 40 à 50% en volume seulement, ce qui correspond à un rapport R des volumes de 0,67 à 1.

Cette technique de concentration, par rapport à la technique d'écrémage décrite précédemment, engendre avec des membranes d'ultrafiltration, de diafiltration ou de dialyse, dont la zone de coupure se situe entre 12 000 et 110 000, de faibles modifications des solutions polysaccharidiques, correspondant à une perte de 0 à 20% en poids de polysaccharides s'accompagnant parfois d'une diminution de la viscosité.

En outre, il faut noter qu'une faible teneur résiduelle en sels affecte les caractéristiques des polysaccharides au cours de l'étape de séchage. En particulier, à concentration égale, les polysaccharides séchés avant dialyse ont des viscosités nettement supérieures aux polysaccharides séchés après dialyse.

La remise en solution des polysaccharides séchés avant dialyse est plus aisée, ce qui peut être avantageux pour leur utilisation comme additif de modifications de rhéologie dont la viscosité d'un milieu liquide notamment dans le domaine agro-alimentaire.

Selon l'invention, la séparation des phases solide et liquide en présence peut être réalisée par centrifugation entre 5 000g et 40 000g et de préférence entre 20 000 et 35 000g à condition que la viscosité du milieu de départ n'excède pas 700 à 800 cPo (à 1,8 par seconde) et que cette centrifugation soit effectuée dans les heures qui suivent l'arrêt de la culture.

Par ailleurs, la viscosité des centrifugats décroît un peu avec l'augmentation du nombre de g, ce qui correspond à l'élimination d'une faible partie des polysaccharides.

Il est aussi possible de réaliser l'étape de séparation des phases solide et liquide par filtration sous vide. La séparation des cultures sur filtres rotatifs à pré-couches de diatomées (ou microalgues) permet l'obtention d'un filtrat clair avec un débit allant de 100 à 400 l/h/m².

Cette technique de séparation solide/liquide, quoique tout fait satisfaisante, permet d'obtenir une teneur en polysaccharides moins élevée que la technique de centrifugation. Une différence maximale de 30% sur la teneur totale en polysaccharides peut être observée.

Le chauffage de la culture selon l'invention avant cette étape de filtration permet d'accroître jusqu'à 30% la teneur du filtrat en polysaccharides. Ceci est dû au fait qu'une partie des polysaccharides de la gangue passe en solution au cours du chauffage.

Selon l'invention, le séchage des polysaccharides est réalisé, pendant 12 à 72 heures et par exemple de 24 à 48 heures, à une température allant de 30 à 60°C, avec notamment le maintien d'une humidité résiduelle d'au moins 5%, en atmosphère ventilée.

D'autres caractéristiques et avantages de l'invention ressortiront mieux de la description qui va suivre, donnée à titre illustratif et non limitatif en référence aux figures annexées, dans lesquelles :
- la figure 1 illustre, schématiquement, les différentes étapes du procédé d'extraction selon l'invention des polysaccharides hydrosolubilisés à partir d'une culture de Porphyridium cruentum, et
- la figure 2 illustre, schématiquement, les différentes étapes du procédé selon l'invention de récupération des polysaccharides péri et exocellulaires produits par une culture de Porphyridium cruentum.

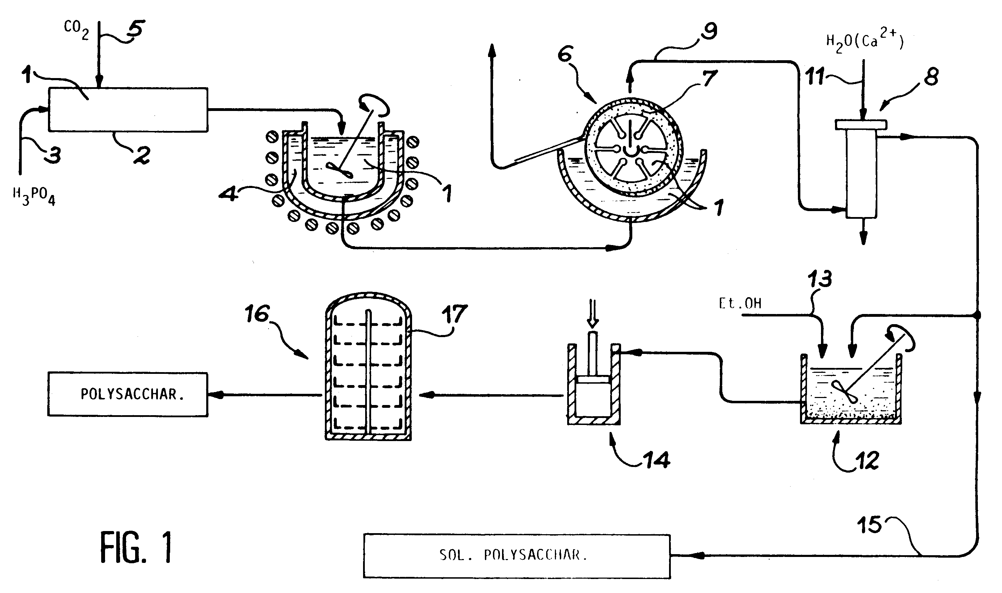

Comme représenté sur la figure 1, la culture 1 de Porphyridium cruentum est réalisée dans un photobioréacteur 2 de 10 m³ environ, contenant comme milieu nourricier de l'eau de mer synthétique enrichie en potassium, phosphore et azote ; le potassium est présent sous forme de chlorure, le phosphore sous forme d'acide orthophosphorique et l'azote sous forme d'urée. Le pH du milieu de culture est régulé entre 6 et 8, comme indiqué en 3 pendant toute la croissance des microalgues, qui dure environ 15 jours, grâce à de l'acide orthophosphorique. L'apport en carbone pour ces microalgues est assuré sous forme de dioxyde de carbone, comme indiqué en 5.

Le photobioréacteur 2 utilisé est en particulier celui décrit dans l'article de C. GUDIN et C. THEPENIER "Bioconversion of solar energy into organic chemicals by microalgae", paru dans Advances in Biotechnological Processes 6, pp. 73-110 de 1986.

La culture 1 est ensuite placée dans un bain-marie 4 porté à 80°C, pendant 30 à 60 minutes, en présence d'une agitation. Le pH n'est alors plus régulé. En outre, l'apport en CO₂ est arrêté.

Comme indiqué en 6, la culture 1 est ensuite filtrée sur un filtre rotatif 7 à pré-couches de diatomées avec un débit voisin de 300 l/h/m² et le rapport diatomées/algues du gâteau de filtration est voisin de 13 pour un filtre de 0,1 m² . Un essai non optimisé sur un filtre de 1,9 m² a donné un rapport de 3,3.

Le filtrat clair 9 obtenu en 6, contenant 70 à 75% en poids des polysaccharides totaux de la culture, est ensuite concentré, comme indiqué en 8, alors que le résidu obtenu en 6 contenant la biomasse cellulaire, c'est-à-dire des microalgues de Porphyridium cruentum peut alors être récupérée et traitée pour l'extraction d'autres produits.

La concentration en 8 du filtrat riche en polysaccharides est réalisée par ultrafiltration associée à une diafiltration avec une membrane minérale de 2 à 50 nm de rayon de pores. Dans ce dernier cas, l'eau circulant, comme indiqué en 11, tangentiellement à la membrane, contient du chlorure de calcium afin d'échanger les ions Na⁺ de la solution polysaccharidique par des ions Ca⁺⁺.

Suivant les cas, des facteurs de concentration volumique de 10 peuvent être obtenus au cours de l'ultrafiltration.

Le filtrat obtenu en 8 est alors précipité, comme indiqué en 12, avec de l'éthanol amené en 13. Le rapport R volume d'alcool/volume du filtrat est égal à 1,0.

Plus de 95% des polysaccharides hydrosolubilisés contenus dans le filtrat issu de 8 sont alors précipités.

Il est aussi possible d'utiliser le résidu obtenu en 6 après un traitement adéquat, tel qu'en pisciculture, en vue de nourrir les poissons ou les mollusques.

Le précipité de polysaccharides, obtenu en 12 est alors soumis à un pressage comme indiqué en 14.

Le produit pressé est ensuite séché, comme indiqué en 16, dans une colonne 17 chauffée à 40°C en présence d'air pendant 48 heures avec maintien d'une humidité résiduelle d'au moins 5% environ. Le produit sec obtenu contient uniquement les polysaccharides qui étaient hydrosolubilisés.

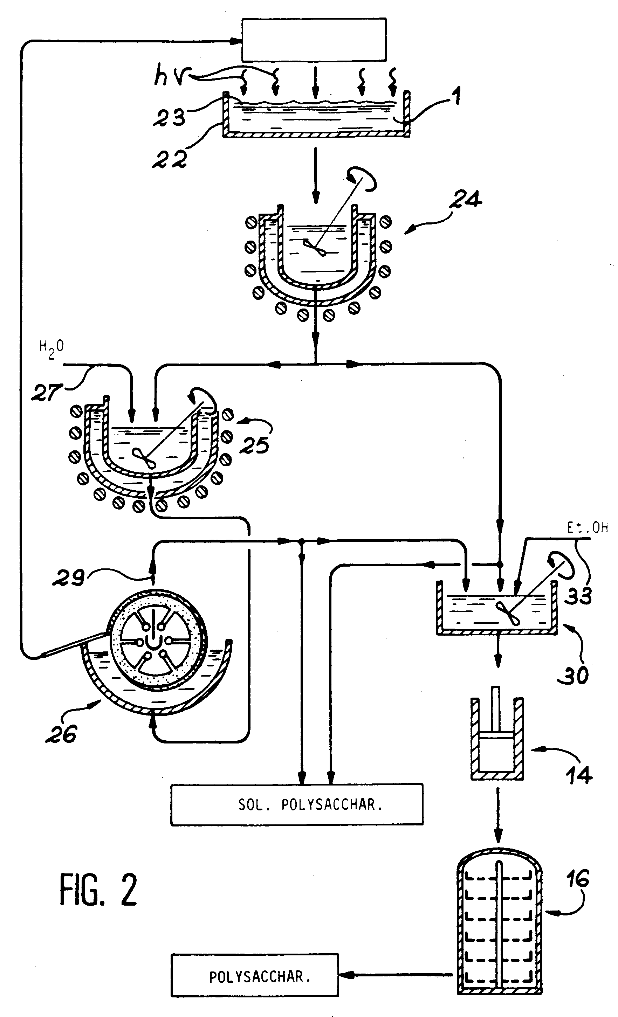

Sur la figure 2, on a représenté schématiquement les différentes étapes de récupération des polysaccharides péri et exocellulaires produits par une culture de Porphyridium cruentum. La culture 1, effectuée dans les mêmes conditions opératoires que décrites en référence à la figure 1, est tout d'abord transférée dans une cuve "plate" 22 de forme parallélépipédique dont le rapport de la surface de base sur le volume est ≧ 10m⁻¹ et dont la hauteur est ≧10cm.

Cette cuve 22 est non agitée, non couverte et laissée dans les conditions naturelles d'intensité lumineuse et de température (correspondant à une intensité lumineuse moyenne de 4000 kcal/m² par jour et à une température de 20 à 40°C), pendant 24 heures. Le pH de la culture n'est plus régulé et l'apport en CO₂ est interrompu.

A la surface de la culture 1, il se forme une crème 23 riche en polysaccharides péri et exocellulaires (5 à 40 g/l de polysaccharides). La crème formée peut être récoltée toutes les 24 heures.

Le produit crémeux 23 récolté est ensuite chauffé dans un bain-marie, comme illustré en 24, à une température de 80°C pendant 1 heure. Ce chauffage est réalisé sous agitation.

Après chauffage du concentrat crémeux et une dilution appropriée, comme indiqué en 25, ce dernier peut être filtré sur un filtre rotatif à pré-couches de diatomées, comme indiqué en 26 et de la même façon que décrite précédemment, en vue d'obtenir les polysaccharides produits par Porphyridium cruentum. Cette filtration sous vide 26 nécessite une dilution préalable du concentrat crémeux, comme représenté en 25 sur la figure.

Il est aussi possible de réaliser la séparation des phases solide et liquide formées, après l'étape de chauffage 24, par centrifugation entre 20 000 et 35 000g.

Le centrifugat ou encore le filtrat 29 obtenu en 26 est alors précipité comme indiqué en 30, avec de l'éthanol arrivant en 33. Le rapport du volume d'éthanol sur le volume du filtrat 29 ou sur le volume du centrifugat est égal à 1,0.

La phase solide obtenue en 26 est quant à elle recyclée en vue de l'extraction d'autres produits (amidon, lipides,...), cette phase solide contenant la majeure partie des microalgues.

Il est possible d'utiliser la culture résiduelle sousjacente à la crème 23 en pisciculture pour l'alimentation des poissons, crustacés.

Le précipité de polysaccharides obtenu en 30 est alors comme précédemment et dans les mêmes conditions, pressé puis séché. Le produit sec obtenu contient les polysaccharides.

On donne ci-après quelques exemples illustrant le procédé de production et d'extraction des polysaccharides produits par Porphyridium cruentum, conformément à l'invention.

### EXEMPLE 1

29 l d'une culture de Porphyridium cruentum contenant 0,7 g/l de polysaccharides n'ayant subi aucune étape de concentration, ont été chauffés 80°C pendant 1 heure puis filtrés à un débit de 60 l/h/m². Le filtrat obtenu contient 2,97 g/l de culture de matières précipitables à l'alcool (et en particulier l'éthanol), soit 85,8% des matières précipitables totales, dont 0,9 g/l de polysaccharides purifiés.

### EXEMPLE COMPARATIF 1

46 l de la même culture que dans l'exemple 1, n'ayant subi aucune étape de concentration ont été filtrés à un débit de 104 l/h/m² . Le filtrat obtenu contient 2,51 g/l de culture de matières précipitables à l'alcool (notamment éthanol), soit 77,5% des matières précipitables totales. Ces matières contiennent 0,7g/l de polysaccharides purifiés (pressés et séchés).

En comparant l'exemple 1 et l'exemple comparatif 1, il ressort clairement que le traitement préalable de la culture à 80°C permet, dans ce cas particulier, une augmentation des matières précipitables à l'alcool dans le filtrat de 18% et une augmentation des polysaccharides purifiés extraits de 30%.

Ceci indique clairement le rôle important joué par l'étape de chauffage conformément à l'invention, avant la précipitation des polysaccharides par l'alcool.

Par matières précipitables l'alcool, il faut comprendre toutes les matières en solution que l'on précipite avec de l'alcool. Ces matières correspondent en particulier aux polysaccharides produits par la microalgue et à une partie des sel présents dans le milieu.

### EXEMPLE 2

Sur un milieu d'eau de mer synthétique contenant 0,6g/l d'urée et 0,75 g/l de chlorure de potassium, on a cultivé dans un photobioréacteur tel que décrit dans l'article de C. GUDIN ci-dessus, des microalgues de Porphyridium cruentum pendant 10 jours. Le pH était régulé à 6,0 avec l'acide orthophosphorique et l'alimentation des microalgues en carbone était faite sous forme de dioxyde de carbone à un débit moyen de 100 l/H/m³ de culture. La température de la culture était de 20-30°C et l'éclairement moyen de 4000 kcal/m² par jour.

La culture obtenue a alors été placée dans une cuve parallélépipédique de 10 cm de hauteur et de 10 m² de surface de base, la longueur valant 10 m et la largeur 1m. La culture a été maintenue en conditions stagnantes (cuve non agitée et non couverte) pendant 24 heures, sans régulation de pH, ni apport en CO₂ , en modifiant la température de la culture et son éclairement. Les résultats obtenus sont donnés dans le tableau I.

**TABLEAU I**

| ECLAIREMENT | TEMPERATURE | CREMAGE |
|---|---|---|
| obscurité | 15°C | Absence de crème |
| " | 35°C | " |
| Lumière artificielle* | 15°C | crème |
| " | 35°C | crème plus intense |

| | | |
|---|---|---|
| *la lumière artificielle avait une intensité de 400 kcal/m²/j. | | |

Le tableau I fait ressortir clairement l'importance de la lumière et d'une température au moins égale à 10°C pour obtenir le phénomène de crémage. Une température supérieure à 40°C entraîne une destruction partielle de la culture ou une inhibition de la production de polysaccharides par les microalgues. De même, une température inférieure à 10°C inhibe le phénomène de crémage.

Avec la cuve ci-dessus de 10m² de surface, en lumière naturelle et 30°C environ, il a été possible d'extraire en 3 écrémages (étalés sur une semaine) 80% des polysaccharides hydrosolubilisés présents, dans seulement 2% du volume de culture placé dans la cuve.

### EXEMPLE 3

1 000 l d'une culture de Porphyridium cruentum, contenant 0,25 g/l de polysaccharides en fin de cycle cultural, sont étalés dans une cuve plate de rapport surface de base/volume égal à 10. Après 24 heures d'exposition en conditions stagnantes et dans les conditions naturelles (lumière solaire et température ambiante), on recueille 11 l de crème contenant 5,34 g/l de polysaccharides, ce qui représente un facteur de concentration massique de 21.

Cette crème est alors diluée de manière appropriée dans de l'eau, chauffée 1 heure à 80°C sous agitation, puis centrifugée à 20 000 g. Le surnageant obtenu contient 8,0 g/l de polysaccharides, ce qui représente une augmentation de 50% de la teneur en polysaccharides par rapport à la teneur obtenue avant l'étape de chauffage.

Les polysaccharides présents dans la phase liquide sont alors précipités par addition d'éthanol à volume égal. Le précipité est pressé et séché à 40°C, en atmosphère ventilée, pendant 48 heures.

### EXEMPLE 4

1,5m³ d'une culture de Porphyridium cruentum contenant 0,21 g/l de polysaccharides en fin de cycle cultural, sont étalés dans une cuve plate de rapport surface/volume égal à 11,3. Après 48 heures d'exposition en conditions naturelles et stagnantes, on recueille 10,3 l de crème contenant 6,8 g/l de polysaccharides. Ceci représente un facteur de concentration massique de 32.

La crème obtenue est alors diluée de manière appropriée dans de l'eau, chauffée 1 heure à 80°C sous agitation, puis centrifugée à 20 000 g. Le surnageant obtenu contient 9,0 g/l de polysaccharides. On obtient ainsi un facteur de concentration massique de 43 au lieu de 32 avant l'étape de chauffage.

Les polysaccharides recueillis sont alors précipités par addition d'éthanol à volume égal. Ce précipité est ensuite pressé puis séché à 40°C, en atmosphère ventilée, pendant 48 heures.

### EXEMPLES 5 à 7

1,5 m³ d'une culture de Porphyridium cruentum, contenant 0,21 g/l de polysaccharides en fin de cycle cultural, sont étalés dans une cuve plate de rapport surface de base/volume égal à 11,3. Après des durées variables d'exposition en conditions naturelles et stagnantes, la crème formée est diluée de manière appropriée dans de l'eau, chauffée pendant 1 heure à 80°C puis centrifugée à 20 000 g. Le surnageant obtenu est alors précipité par addition de 1 volume d'éthanol. Le précipité obtenu est pressé puis séché à 40°C, en atmosphère ventilée, pendant 48 heures. Les résultats obtenus sont donnés dans le tableau II, ci-après.

**TABLEAU II**

| Ex. | Temps de repos avant la récolte de la crème (jour) | Volume du concentrat crémeux récupéré (l) | Concentration en polysaccharides dans le concentrat crémeux (g/l) | Facteur de concentration | Rendement de la récolte (% de polysaccharides exocellulaires) |
|---|---|---|---|---|---|
| 5 | 3 | 15 | 9,1 | 43 | 43,5 |
| 6 | 2 | 10,3 | 9 | 43 | 29,5 |
| 7 | 1 | 7,0 | 5 | 24 | 11 |

Ce tableau II indique clairement que la quantité de crème riche en polysaccharides, et donc celle des polysaccharides , augmente avec la durée d'exposition de la culture initiale à la lumière solaire, en conditions naturelles et stagnantes.

### EXEMPLES COMPARATIFS 2 à 4

5 m³ d'une culture de Porphyridium cruentum contenant 0,65 g/l de polysaccharides en fin de cycle cultural ont été étalés dans une cuve de rapport surface de base/volume égal à 0,70. La culture a été maintenue en conditions naturelles et stagnantes pendant des durées variables. La crème obtenue a alors été diluée de manière appropriée dans de l'eau, chauffée pendant 1 heure à 80°C puis centrifugée à 20 000 g. Le surnageant obtenu a été précipité par de l'éthanol à volume égal puis pressé et enfin séché à 40°C, en atmosphère ventilée, pendant 48 heures. Les résultats sont donnés dans le tableau III ci-après.

**TABLEAU III**

| Ex. comparatifs | Temps de repos avant la récolte de la crème (jour) | Volume du concentrat crémeux récupéré (l) | Concentration en polysaccharides dans le concentrat crémeux (g/l) | Facteur de concentration | Rendement de la récolte (% de polysaccharides exocellulaires) |
|---|---|---|---|---|---|
| 2 | 4 | 7 | 5,83 | 9 | 1,3 |
| 3 | 2 | 3,2 | 12,3 | 19 | 1,2 |
| 4 | 1 | 2,1 | 8,05 | 12,5 | 0,5 |

Du tableau III, il ressort aussi que plus la durée d'exposition en conditions naturelles de la culture est longue, plus la quantité de polysaccharides produite est importante. Toutefois, une exposition supérieure à 4 jours ne permet pas d'augmenter de façon considérable le rendement de la production.

En outre, la comparaison de ces exemples comparatifs 2-4 avec les exemples 5-7 indique clairement l'importance jouée sur le phénomène de crémage, et donc sur la production de polysaccharides, du rapport surface/volume de la cuve exposée à la lumière solaire, en conditions naturelles et stagnantes.

En effet, pour un rapport de 0,7, le rendement est égal à 1,3 au bout de 4 jours d'exposition, alors qu'il est égal à 43,5 au bout de 3 jours avec un rapport de 11,3.

### EXEMPLE 8

Une culture de Porphyridium cruentum contenant 0,21 g/l de polysaccharides en fin de cycle cultural a été étalée dans une cuve plate de rapport surface/volume égal à 11 pendant 6 jours, en conditions stagnantes et naturelles d'exposition solaire et de température. La crème obtenue a ensuite été diluée de manière appropriée dans de l'eau, chauffée 1 heure à 80°C puis centrifugée à 20 000 g. Le surnageant obtenu a été précipité par addition d'un volume égal d'éthanol. Le précipité a été pressé puis séché à 40°C en atmosphère ventilée pendant 48 heures.

### EXEMPLE COMPARATIF 5

Une culture de Porphyridium cruentum contenant 0,65 g/l de polysaccharides en fin de cycle cultural a été étalée dans une cuve de rapport surface/volume égal à 0,6 pendant 7 jours, en conditions naturelles et stagnantes. La crème obtenue a été traitée de la même façon que dans l'exemple 8.

Les résultats obtenus sont donnés dans le tableau IV ci-après.

**TABLEAU IV**

| | Volume total du concentrat crémeux récupéré (l) | Concentration en polysaccharides de la crème (g/l) | Facteur de concentration | Rendement total de la récolte (% de polysaccharides exocellulaires) |
|---|---|---|---|---|
| Exemple comparatif 4 | 12,3 | 8,13 | 12,5 | 3 |
| Exemple 7 | 32,3 | 8,28 | 39 | 84 |

La comparaison de l'exemple 8 et de l'exemple comparatif 5 montre encore l'importance jouée sur la production de crème riche en polysaccharides du rapport surface/volume de la cuve.

## Revendications

1. Procédé de production et d'extraction de polysaccharides à partir d'une culture de la microalgue Porphyridium cruentum, comprenant une étape de concentration (8, 23) des polysaccharides péri- et exocellulaires produits par la microalgue, une étape de séparation des phases solide et liquide en présence en vue du recyclage de la microalgue, une étape de chauffage (4, 24) à une température allant de 60 à 100°C, une étape de précipitation (12, 30) des polysaccharides présents dans la phase liquide et une étape de séchage (16) du précipité obtenu.

2. Procédé selon la revendication 1, caractérisé en ce qu'une étape de pressage (14) est prévue entre les étapes de précipitation (12, 30) et de séchage (16) du précipité.

3. Procédé selon la revendication 1 ou 2, caractérisé en ce que l'étape de précipitation (12, 30) des polysaccharides est effectuée par addition d'alcool dans un rapport volume d'alcool/volume de la solution au plus égal à 1.

4. Procédé selon la revendication 3, caractérisé en ce que le rapport volume d'alcool/volume de la solution va de 0,5 à 1.

5. Procédé selon l'une quelconque des revendications 1 à 4, caractérisé en ce que le chauffage (4, 24) est effectué à une température de l'ordre de 80°C.

6. Procédé selon l'une quelconque des revendications 1 à 5, caractérisé en ce que la concentration des polysaccharides consiste à placer la culture dans un photoréacteur (22) exposé à la lumière solaire en conditions stagnantes, entraînant ainsi la formation d'une crème (23) riche en polysaccharides, et à récupérer ladite crème par écrémage.

7. Procédé selon l'une quelconque des revendications 1 à 6, caractérisé en ce qu'il consiste essentiellement à :
- placer la culture dans un photobioréacteur exposé à la lumière solaire en conditions stagnantes entraînant la formation d'une crème (23) riche en polysaccharides péri et exocellulaires,
- récupérer la crème par écrémage,
- chauffer (24) le concentrat crémeux,
- séparer (26) les phases liquide et solide du concentrat,
- précipiter (30) les polysaccharides présents dans la phase liquide,
- presser (14) le précipité de polysaccharides, et
- sécher (16) le précipité pressé.

8. Procédé selon la revendication 6 ou 7, caractérisé en ce que la culture est maintenue dans le photoréacteur (22) à une température allant de 10 à 40°C pendant 12 à 72 heures.

9. Procédé selon l'une quelconque des revendications 6 à 8, caractérisé en ce qu'il comprend une étape de solubilisation (25) de la crème formée (23) avant d'effectuer l'étape de précipitation (30).

10. Procédé selon l'une quelconque des revendications 6 à 9, caractérisé en ce que le photoréacteur présente la forme d'une cuve plate (22) dont le rapport surface/volume est au moins égal à 10 et dont l'épaisseur est au moins égale à 10 cm.

11. Procédé selon l'une quelconque des revendications 1 à 5, caractérisé en ce que la concentration (8) des polysaccharides comprend une étape d'ultrafiltration éventuellement associée à une diafiltration.

12. Procédé selon l'une quelconque des revendications 1 à 5, caractérisé en ce qu'il consiste essentiellement à :
- chauffer (4) la culture de la microalgue,
- séparer (6) les phases solide et liquide de la culture chauffée,
- concentrer (8) les polysaccharides péri et exocellulaires présents dans la phase liquide par ultrafiltration éventuellement associée à une diafiltration,
- précipiter (12) les polysaccharides présents dans le filtrat,
- presser (14) le précipité de polysaccharides, et
- sécher (16) le précipité pressé.

13. procédé selon t'une quelconque des revendications 1 à 12, caractérisé en ce que le précipité est séché en atmosphère ventilée à une température allant de 30 à 60°C.

14. Procédé selon l'une quelconque des revendications 1 à 13, caractérisé en ce que le séchage est effectué avec une humidité résiduelle d'au moins 5%.

15. Procédé selon l'une quelconque des revendications 1 à 14, caractérisé en ce que l'on sépare les phases solide et liquide par centrifugation entre 5 000g et 40 000g.

16. Procédé selon l'une quelconque des revendications 1 à 14, caractérisé en ce que l'on sépare les phases solide et liquide par filtration (6, 26) sous vide.

## Claims

1. Process for the production and traction of polysaccharides from a culture of the microalga porphyridium cruentum, comprising a stage (8, 23) of concentrating pericellular and exocellular polysaccharides produced by the microalga, a stage of separating the solid and liquid phases present with a view to the recycling of the microalga, a heating stage (4, 24) at a temperature between 60 and 100°C, a stage (12, 30) of precipitating the polysaccharides present in the liquid phase and a stage (16) of drying the precipitate obtained.

2. Process according to claim 1, characterized in that there is a pressing stage (14) between the precipitation stage (12, 30) and the drying stage (16) of the precipitate.

3. Process according to claim 1 or 2, characterized in that the polysaccharide precipitation stage (12, 30) is carried out by adding alcohol in a ratio of the alcohol volume to the solution volume of at the most 1.

4. Process according to claim 3, characterized in that the alcohol volume/ solution volume ratio is 0.5 to 1.

5. Process according to any one of the claims 1 to 4, characterized in that the heating stage (4, 24) is performed at approximately 80°C.

6. Process according to any one of the claims 1 to 5, characterized in that the concentration of the polysaccharides consists of placing the culture in a photoreactor (22) exposed to sunlight under stagnant conditions, thus leading to the formation of a polysaccharide-rich cream (23) and to the recovery of said cream by skimming.

7. Process according to any one of the claims 1 to 6, characterized in that it essentially consists of placing the culture in a photobioreactor exposed to sunlight under stagnant conditions leading to the formation of a cream (23) rich in pericellular and exocellular polysaccharides, recovering the cream by skimming, heating (24) the creamy concentrate, separating (26) the liquid and solid phases from the concentrate, precipitating (30) the polysaccharides present in the liquid phase, pressing (14) the polysaccharide precipitate and drying (16) the pressed precipitate.

8. Process according to claim 6 or 7, characterized in that the culture is kept in the photoreactor (22) at a temperature between 10 and 40°C for 12 to 72 hours.

9. Process according to any one of the claims 6 to 8, characterized in that it comprises a stage (25) of solubilizing the cream formed (23) before carrying out the precipitation stage (30).

10. Process according to any one of the claims 6 to 9, characterized in that the photoreactor is shaped like a shallow vessel (22), whose surface/volume ratio is at least equal to 10 and whose thickness is at least equal to 10 cm.

11. Process according to any one of the claims 1 to 5, characterized in that the concentration (8) of the polysaccharides comprises an ultrafiltration stage, optionally associated with a diafiltration.

12. Process according to any one of the claims 1 to 5, characterized in that it essentially consists of heating (4) the microalga culture, separating (6) the solid and liquid phases from the heated culture, concentrating (8) the pericellular and exocellular polysaccharides present in the liquid phase by an ultrafiltration, optionally associated with a diafiltration, precipitating (12) the polysaccharides present in the filtrate, pressing (14) the polysaccharide precipitate and drying (16) the pressed precipitate.

13. Process according to any one of the claims 1 to 12, characterized in that the precipitate is dried in a ventilated atmosphere at a temperature of 30 to 60°C.

14. Process according to any one of the claims 1 to 13, characterized in that drying takes place with a residual moisture of at least 5%.

15. Process according to any one of the claims 1 to 14, characterized in that the liquid and solid phases are separated by centrifuging at between 5000 and 40,000 g.

16. Process according to any one of the claims 1 to 14, characterized in that the solid and liquid phases are separated by vacuum filtration (6, 26).

## Patentansprüche

1. Verfahren zur Herstellung und Extraktion von Polysacchariden aus einer Kultur der Mikroalge Porphyridium cruentum, umfassend einen Schritt des Konzentrierens (8, 23) der von der Mikroalge gebildeten peri- und exozellulären Polysaccharide, einen Schritt des Trennens der vorhandenen festen und flüssigen Phase mit dem Ziel, die Mikroalge rückzuführen, einen Schritt der Erwärmung (4, 24) auf eine Temperatur zwischen 60 und 100°C, einen Schritt des Ausfällens (12, 30) der vorhandenen Polysaccharide und einen Schritt des Trocknens (16) des erhaltenen Niederschlags.

2. Verfahren nach Anspruch 1, dadurch gekennzeichnet, daß ein Schritt des Auspressens (14) zwischen den Schritten des Fällens (12, 30) und des Trocknens (16) des Niederschlags vorgesehen ist.

3. Verfahren nach Anspruch 1 oder 2, dadurch gekennzeichnet, daß der Schritt des Ausfällens (12, 30) der Polysaccharide durch Zugabe von Alkohol in einem Verhältnis von Alkoholvolumen zu Lösungsvolumen höchstens gleich 1 durchgeführt wird.

4. Verfahren nach Anspruch 3, dadurch gekennzeichnet, daß das Verhältnis von Alkoholvolumen zu Lösungsvolumen zwischen 0,5 und 1 liegt.

5. Verfahren nach einem der Ansprüche 1 bis 4, dadurch gekennzeichnet, daß das Erwärmen (4, 24) auf eine Temperatur der Größenordnung 80°C erfolgt.

6. Verfahren nach einem der Ansprüche 1 bis 5, dadurch gekennzeichnet, daß das Konzentrieren der Polysaccharide darin besteht, die Kultur in einen Photoreaktor (22) zu bringen, der dem Sonnenlicht unter ruhenden Bedingungen ausgesetzt ist, was zur Bildung einer polysaccharidreichen Creme (23) führt, und genannte Creme durch Abschöpfen zu gewinnen.

7. Verfahren nach einem der Ansprüche 1 bis 6, dadurch gekennzeichnet, daß es im wesentlichen darin besteht,
- die Kultur in einen Photobioreaktor zu bringen, der dem Sonnenlicht unter ruhenden Bedingungen ausgesetzt ist, was zur Bildung einer an peri- und exozellulären Polysacchariden reichen Creme (23) führt,
- die Creme durch Abschöpfen zu gewinnen,
- das cremige Konzentrat zu erhitzen (24),
- die flüssige und die feste Phase des Konzentrats zu trennen (26),
- die in der flüssigen Phase vorhandenen Polysaccharide auszufällen (30),
- den Polysaccharidniederschlag auszupressen (14) und
- den ausgepreßten Niederschlag zu trocknen (16).

8. Verfahren nach Anspruch 6 oder 7, dadurch gekennzeichnet, daß die Kultur (22) 12 bis 72 Stunden lang bei einer Temperatur zwischen 10 und 40°C im Photoreaktor gehalten wird.

9. Verfahren nach einem der Ansprüche 6 bis 8, dadurch gekennzeichnet, daß es vor Durchführung des Fällungsschrittes (30) einen Schritt des Auflösens (25) der gebildeten Creme (23) umfaßt.

10. Verfahren nach einem der Ansprüche 6 bis 9, dadurch gekennzeichnet, daß der Photoreaktor die Gestalt einer flachen Wanne (22) besitzt, bei der das Verhältnis von Oberfläche zu Volumen wenigstens 10 ist und deren Dicke wenigstens gleich 10 cm ist.

11. Verfahren nach einem der Ansprüche 1 bis 5, dadurch gekennzeichnet, daß das Konzentrieren (8) der Polysaccharide einen Schritt der Ultrafiltration, gegebenenfalls verbunden mit einer Diafiltration, umfaßt.

12. Verfahren nach einem der Ansprüche 1 bis 5, dadurch gekennzeichnet, daß es im wesentlichen darin besteht,
- das Kultur der Mikroalge zu erhitzen (4),
- die flüssige und die feste Phase der erhitzten Kultur zu trennen (6),
- die in der flüssigen Phase vorhandenen peri- und exozellulären Polysaccharide durch Ultrafiltration, gegebenenfalls verbunden mit einer Diafiltration, zu konzentrieren (8),
- die im Filtrat vorhandenen Polysaccharide auszufällen (12),
- den Polysaccharidniederschlag auszupressen (14) und
- den ausgepreßten Niederschlag zu trocknen (16).

13. Verfahren nach einem der Ansprüche 1 bis 12, dadurch gekennzeichnet, daß der Niederschlag in belüfteter Atmosphäre bei einer Temperatur von 30 bis 60°C getrocknet wird.

14. Verfahren nach einem der Ansprüche 1 bis 13, dadurch gekennzeichnet, daß die Trocknung mit einer Restfeuchtigkeit von mindestens 5% ausgeführt wird.

15. Verfahren nach einem der Ansprüche 1 bis 14, dadurch gekennzeichnet, daß man die feste und die flüssige Phase durch Zentrifugieren zwischen 5 000g und 40 000g trennt.

16. Verfahren nach einem der Ansprüche 1 bis 14, dadurch gekennzeichnet, daß man die feste und die flüssige Phase durch Filtration (6, 26) unter Vakuum trennt.
